# FASCICULE DE BREVET EUROPEEN

(11) **EP 2 731 966 B1**
(45) Date de publication et mention de la délivrance du brevet: **22.02.2017**
(21) Numéro de dépôt: 12733726.9
(22) Date de dépôt: 11.07.2012
(51) Int. Cl.: C07K 16/06, C07K 1/36

(54) **PROCEDE DE PREPARATION D'UN CONCENTRE D'IMMUNOGLOBULINES POLYVALENTES**
VERFAHREN ZUR HERSTELLUNG EINES POLYVALENTEN IMMUNOGLOBULINKONZENTRATS
METHOD FOR PREPARING A POLYVALENT-IMMUNOGLOBULIN CONCENTRATE

(30) Priorité: 11.07.2011 FR 1156285
(43) Date de publication de la demande: 21.05.2014
(73) Titulaire: Laboratoire Français du Fractionnement et des Biotechnologies, 91940 Les Ulis (FR)
(72) Inventeur: CHTOUROU, Abdessatar, F-78990 Elancourt (FR); BATAILLE, Damien, F-91540 Ormoy (FR); MICHAUX, Georges, F-78470 St Rémy Les Chevreuse (FR)
(74) Mandataire: Pontet Allano & Associes
(86) Numéro de dépôt international: PCT/EP2012/063549
(87) Numéro de publication internationale: WO 2013/007740

(56) Documents cités:
- EP-A1- 1 385 886
- WO-A2-2007/077365
- WO-A2-2011/131786
- US-A- 5 075 425
- US-A1- 2003 152 966
- US-B1- 7 041 798
- BUCHACHER ANDREA ET AL: "Purification of intravenous immunoglobulin G from human plasma - aspects of yield and virus safety", BIOTECHNOLOGY JOURNAL, vol. 1, no. 2, 1 février 2006 (2006-02-01) , pages 148-163, XP009141576, WILEY-VCH VERLAG, WEINHEIM, DE ISSN: 1860-6768
- ANONYME: "AVIS: CLAIRYG 50 mg/ml, solution pour perfusion", HAS - HAUTE AUTORITÉ DE SANTÉ , 10 février 2010 (2010-02-10), pages 1-10, XP002667349, Extrait de l'Internet: URL:http://www.has-sante.fr/portail/upload /docs/application/pdf/2010-03/clairyg_-_ct -7523.pdf [extrait le 2012-01-17]
- LEBING W ET AL: "PROPERTIES OF A NEW INTRAVENOUS IMMUNOGLOBULIN (IGIV-C, 10%) PRODUCED BY VIRUS INACTIVATION WITH CAPRYLATE AND COLUMN CHROMATOGRAPHY", VOX SANGUINIS, S. KARGER AG, BASEL, CH, vol. 84, no. 3, 1 avril 2003 (2003-04-01), pages 193-201, XP001197282, ISSN: 0042-9007, DOI: 10.1046/J.1423-0410.2003.00285.X
- RADOSEVICH M ET AL: "Intravenous immunoglobulin G: Trends in production methods, quality control and quality assurance", VOX SANGUINIS, S. KARGER AG, BASEL, CH, vol. 98, no. 1, 1 janvier 2010 (2010-01-01), pages 12-28, XP009141581, ISSN: 0042-9007
- "Immunoglobuline humaine normale pour administration par voie intraveineuse" In: "Pharmacopée Européenne 7.5", 1 January 2012 (2012-01-01) pages 4965-4966,
- 'European Pharmacopoeia 5.0', partie 2.6.17 Bd. 'Test for anticomplenetary activity of immunoglobulin', pages 170 - 172

## Description

La présente invention concerne un procédé de préparation d'un concentré d'immunoglobulines polyvalentes en vue d'une utilisation thérapeutique.

Les immunoglobulines (Ig) sont synthétisées par les lymphocytes B et les plasmocytes, qui se distribuent dans le plasma, les liquides extravasculaires et les sécrétions. Elles sont divisées en 5 catégories, isotypes ou classes, sur la base de leur structure protéique: IgG, IgA, IgM, IgE et IgD. Leur répartition naturelle dans le corps humain est la suivante: IgG : 75 %, IgA : 20 %, IgM : 5 % et < 1 % pour IgE et Ig D. Il y a normalement de 8 à 15 g d'IgG par litre de plasma. Dans le plasma, la demi-vie des immunoglobulines circulantes de classe IgG, est d'environ 21 jours, celle des IgA, IgM, IgD, IgE est inférieure à 7 jours.

Les immunoglobulines polyvalentes d'origine humaine et pharmaceutiquement utilisées sont composées à 97 % d'IgG correspondant à la présence d'une grande diversité d'anticorps contre divers agents infectieux.

L'utilisation de fractions de plasma humain enrichies en immunoglobulines polyvalentes pour le traitement de diverses infections ou déficiences congénitales est connue pour son effet thérapeutique. Les immunoglobulines polyvalentes ou normales sont administrées pour corriger un déficit immunitaire primitif et certains déficits secondaires (leucémies, myélomes ou infections récidivantes). Elles sont à dissocier des anticorps spécifiques (Anticorps cytotoxiques anti-rhésus ou anti-D). Elles peuvent être prescrites à doses très élevées, jusqu'à 2 g/kg/jour, pour freiner l'évolution de certaines maladies, dont la physiopathologie est mal connue, mais comporte une composante de type immunitaire. L'administration d'immunoglobulines polyvalentes a montré son efficacité, au moins transitoire, dans le traitement de la thrombocytopénie d'origine immunologique, appelée aussi purpura thrombogénique idiopathique. Les immunoglobulines polyvalentes peuvent également avoir un effet bénéfique dans le traitement du syndrome de Guillain-Barré, des polyneuropathies démyélinisantes, de la sclérose en plaques, du syndrome myasthénique de Lambert-Eaton, des dermatomyosites.

Les immunoglobulines polyvalentes contiennent diverses molécules si bien que leur mécanisme d'action est complexe : en intervenant à plusieurs niveaux, elles modifient l'équilibre immunitaire du patient et peuvent ainsi avoir un effet bénéfique.

Plusieurs procédés de préparation de concentrés d'immunoglobulines polyvalentes sont déjà connus de l'homme du métier. Le procédé de préparation le plus connu comprend plusieurs étapes de précipitation à l'éthanol (Cohn et al. 1946, J. Am. Chem. Soc. 68, 459).

Le brevet EP 0703 922 (Laboratoire Français du Fractionnement et des Biotechnologies, « Concentré d'immunoglobulines G à usage thérapeutique et procédé de production dudit concentré ») décrit en particulier un procédé de production d'un concentré d'immunoglobulines G à partir de plasma humain ou de surnageant de plasma cryoprécipité. Ce procédé ne comprend pas de précipitation à l'éthanol et comprend une succession d'étapes de chromatographie et une étape d'inactivation virale par solvant/détergent. Cette méthode ne permet que de donner un rendement relativement faible, à cause de la succession d'étapes de chromatographie, le procédé décrit incluant en effet deux cycles d'échangeur anionique et cationique en tandem ainsi que deux étapes d'ultrafiltration.

Le brevet EP 1 385 886 (Laboratoire Français du Fractionnement et des Biotechnologies, « Procédé de préparation de concentrés d'immunoglobulines humaines à usage théarapeutique ») décrit un procédé de préparation de concentrés d'immunoglobulines humaines comprenant une prépurification par précipitation de contaminants lipidiques et protéiques et une étape unique de chromatographie effectuée sur un échangeur d'anions à pH alcalin. Cette méthode permet d'obtenir jusqu'à 4g d'immunoglobulines par litre de plasma initialement traité.

La publication de Tanaka K. et al., 2000 ("High quality human immunoglobulin G purified from Cohn fractions by liquid chromatography » Braz J Med Bio Res 2000, 33(1): 27-30) décrit un procédé de purification d'immunoglobulines G à partir de fractions « I+II+II » et « II+III » obtenues selon la méthode de Cohn, par séparation par chromatographie sur trois types de gels, deux gels échangeurs d'ions (Q-Sépharose FF et CM-Sépharose FF) et un gel filtration (Séphacryl S-300 HR). Ce procédé permet d'obtenir jusqu'à 4.3 ± 0.2 g d'immunoglobulines G par litre de plasma.

Le document WO2007/077365 décrit un concentré d'immunoglobulines G à usage thérapeutique dans lequel les teneurs respectives en anticorps anti-A et anti-B sont conformes à un résultat négatif au test de Coombs indirect in vitro. Ce concentré d'IgG présente en outre une teneur d'IgG polyréactives comprise entre 0,01% et 0,1%, en particulier entre 0,07 et 0,1%, par rapport à la teneur totale en IgG. Le procédé d'obtention du concentré d'IgG du document WO2007/077365, comprenant une étape de préparation d'un concentré d'IgG par fractionnement éthanolique et/ou par séparation chromatographique, associant une étape d' inactivation virale, une étape de chromatographie d' immunoaffinité par percolation dudit concentré d' IgG sur un mélange de supports dont les matrices sont greffées de groupes oligosaccharides antigéniquement similaires aux groupes sanguins A et B, et une étape de filtration d'élimination de virus et/ou de particules de taille supérieure à 20 nm.

Néanmoins, les indications thérapeutiques des immunoglobulines s'étant multipliées, il existe un besoin important d'augmenter le rendement de la production d'immunoglobulines, tout en s'assurant que le produit final présente une pureté élevée et soit dépourvu de contaminants viraux.

Par conséquent, la présente invention a pour objet un procédé de préparation d'un concentré d'immunoglobulines polyvalentes humaines avec un rendement supérieur par rapport aux procédés connus de l'homme du métier.

L'invention concerne ainsi un procédé de préparation d'un concentré d'immunoglobulines polyvalentes humaines à partir d'une solution initiale de plasma sanguin ou d'une fraction de plasma enrichie en immunoglobulines, caractérisé en ce qu'il comprend :
(a) une étape d'élimination des contaminants protéiques par l'acide caprylique pour obtenir une solution dépourvue en protéases, dans laquelle la concentration en acide caprylique utilisée va de 0,5 à 1,5%, exprimée en gramme d'acide caprylique par volume de solution à traiter,
(b) une étape de clarification à pH acide par filtration en profondeur,
(c) suivie d'une étape de chromatographie en lit fluidisé de la solution dépourvue en protéases,
ledit procédé permettant d'obtenir un concentré d'immunoglobulines polyvalentes humaines avec un rendement supérieur à 4,5 g d'immunoglobulines/litre de solution initiale de plasma sanguin ou de fraction de plasma enrichie en immunoglobulines.

La solution obtenue à l'issue de l'étape (a) d'élimination des contaminants protéiques par l'acide caprylique du procédé de l'invention possède une concentration en protéases conforme à la norme 2.6.15 de la Pharmacopée Européenne 7.5 Edition 01/2012 :0918, laquelle est au maximum de 35 UI/mL calculée par rapport à une dilution de la préparation à examiner contenant 30g/L d'immunoglobulines.

L'invention repose sur la constatation que la combinaison d'une étape d'élimination des contaminants protéiques par l'acide caprylique avec une étape de chromatographie en lit fluidisé permet d'augmenter de façon surprenante le rendement de la production d'immunoglobulines.

L'invention comprend la combinaison d'une étape ménagée d'élimination des contaminants protéiques par l'acide caprylique à pH acide suivie d'une clarification à pH acide du surnageant obtenu à l'issue de l'étape de traitement par l'acide caprylique permettant la fixation des immunoglobulines sur un gel de chromatographie mis en oeuvre dans une colonne fluidisée (gel de type échangeur anionique et/ou type affinité et/ou de type « pseudo-affinité », de préférence de type « mode-mixte »), ce qui permet d'augmenter le rendement de la production d'immunoglobulines polyvalentes. De façon préférentielle, la productivité peut être augmentée si les conditions d'élution des immunoglobulines polyvalentes du gel de chromatographie permettent une compatibilité directe avec les étapes suivantes du procédé sans étape de formulation par des techniques de type dialyse ou ultrafiltration. De façon particulière, le procédé selon l'invention permet d'obtenir un gain de productivité de 0,25 % d'immunoglobulines polyvalentes par litre de plasma et un produit final ayant une pureté élevée (99 %).

Le procédé de l'invention comprend entre l'étape d'élimination de contaminants protéiques par l'acide caprylique (a) et l'étape de chromatographie en lit fluidisé (b), une étape de clarification à pH acide par filtration en profondeur.

Les étapes d'élution seront avantageusement définies afin d'extraire sélectivement les immunoglobulines de type G (IgG).

L'invention concerne également un procédé de préparation d'un concentré d'immunoglobulines polyvalentes humaines à partir d'une solution initiale de plasma sanguin ou d'une fraction de plasma enrichie en immunoglobulines, caractérisé en ce qu'il comprend :
(a) une étape d'élimination des contaminants protéiques par l'acide caprylique à pH acide pour obtenir une solution dépourvue en protéases,
(b) une étape de clarification à pH acide,
(c) une étape de chromatographie en lit fluidisé de la solution dépourvue en protéases,
(d) une élution dans un tampon d'élution des immunoglobulines.

De façon particulière, l'étape (b) de clarification à pH acide est réalisée par une filtration en profondeur se basant sur une porosité la plus élevée possible mais autorisant le passage du surnageant clarifié sur une colonne en lit fluidisé.

La solution obtenue à l'issue de l'étape (a) d'élimination des contaminants protéiques par l'acide caprylique du procédé de l'invention possède une concentration en protéases conforme à la norme 2.6.15 de la Pharmacopée Européenne 7.5 Edition 01/2012 :0918, laquelle est au maximum de 35 UI/mL calculée par rapport à une dilution de la préparation à examiner contenant 30 g/L d'immunoglobulines.

L'invention concerne également le procédé tel que décrit ci-dessus et intégrant après l'étape (b) de clarification à pH acide une étape de remontée de pH, correspondant au conditionnement des immunoglobulines polyvalentes permettant la fixation de ces dernières sur un gel de chromatographie mis en oeuvre en mode fluidisé.

De façon surprenante, le concentré d'immunoglobulines polyvalentes humaines obtenu à l'issue du procédé de l'invention présente une activité anti-complémentaire inférieure à 30%, laquelle est déterminée en référence à la méthode indiquée dans la Pharmacopée européenne 7.5, édition 01/2012 :0918, paragraphe 2.6.17.

On entend par « immunoglobulines polyvalentes » dans le cadre de l'invention, des immunoglobulines entières, ou des fragments d'immunoglobulines, tels que F(ab')2 ou F(ab) et toute fraction intermédiaire obtenue au cours du procédé de fabrication.

On entend par « fraction de plasma », toute solution sanguine obtenue après un traitement qui permet de séparer ou fractionner du plasma sanguin humain naturel, notamment toute fraction intermédiaire obtenue au cours du procédé de préparation d'un concentré d'immunoglobulines polyvalentes.

On entend par « fraction de plasma enrichie en immunoglobulines », une fraction de plasma contenant un taux d'immunoglobulines supérieur à celui contenu dans du plasma humain naturel.

On entend par précipité « I+II+II » ou « II+III », un précipité obtenu à partir de plasma sanguin fractionné à l'éthanol selon la méthode de Cohn (Cohn et al. 1946, J. Am. Chem. Soc. 68, 459) ou de Kistler et Nistschmann (1962, Vox Sang. 7, 414).

On entend par « contaminants protéiques » toutes les protéines autres que les immunoglobulines de type G, par exemple et de façon non limitative, les immunoglobulines de type A, E ou M ainsi que les autres protéines plasmatiques telles que, de façon non limitative :
- l'albumine, la transferrine, l'α2-macroglobuline, le plasminogène, le fibrinogène etc.,
- les facteurs de la coagulation tels que, de façon non limitative, le FX, FXI, ou le FXII,
- les protéases telles, de façon non limitative, la kallikréine, la prékallikréine, ou l'activateur de la prékallikréine, et
- les hémagglutinines anti-A et -B.

On entend par « chromatographie en lit fluidisé » : une technique qui par application d'un débit dans le sens inverse de la gravité sur des billes de chromatographie de densité élevée permet de ménager un espace entre celles-ci permettant ainsi le passage dans la colonne de chromatographie d'une solution faiblement clarifiée sans engendrer de colmatage du flux de solution. Les chromatographies en lit fluidisé peuvent être des chromatographies de type échange d'ions, de type affinité, de type « pseudo-affinité », ou de type « mode-mixte » (c'est-à-dire à la fois échange d'ions et pseudo-affinité).

De façon particulière, la chromatographie en lit fluidisé est réalisée avec le gel UpFront IVIG de type « mode-mixte » comprenant la présence sur les billes d'un ligand chimique combinant des charges électrostatiques et des groupements hydrophobes conférant une pseudo-affinité spécifique pour les immunoglobulines.

La concentration d'acide caprylique utilisée dans le procédé selon l'invention est ménagée, c'est-à-dire que la précipitation des contaminants est réalisée avec une concentration finale en acide caprylique suffisamment faible de manière à ne pas piéger d'immunoglobulines polyvalentes lors de la précipitation des autres constituants de la solution. La concentration en acide caprylique va de 0,5 à 1,5 %, avantageusement de 0,8 à 1,2 %, particulièrement de 0,9 à 1,1 % et plus particulièrement est égale à 1 % (le pourcentage en acide caprylique correspond à des grammes d'acide caprylique par volume de solution à traiter).

Dans un mode de réalisation, l'étape d'élimination des contaminants protéiques par l'acide caprylique se déroule à un pH compris entre 4,3 et 4,9, de préférence compris entre 4,6 et 4,8.

La demanderesse a constaté de façon surprenante que la concentration d'acide caprylique mise en oeuvre dans le procédé de préparation d'un concentré d'immunoglobulines polyvalentes selon l'invention influence directement le rendement du procédé lorsque ce dernier comprend une étape ultérieure de clarification. Si la concentration en acide caprylique est trop faible, la précipitation sera imparfaite (en particulier une partie importante de lipides reste en solution) et l'étape de clarification sera très difficile à mettre en oeuvre. Par contre, si la concentration en acide caprylique est trop forte, les immunoglobulines polyvalentes seront piégées dans le précipité formé et ne seront plus disponibles dans le surnageant après une clarification.

Dans un mode de réalisation, une étape de clarification à pH acide est ajoutée entre l'étape d'élimination des contaminants protéiques par l'acide caprylique et l'étape de chromatographie en lit fluidisé. Cette étape de clarification à pH acide est réalisée à une valeur de pH comprise entre 4.3 et 4.9, de préférence entre 4.4 et 4.8 et de manière préférée à un pH compris entre 4.6 et 4.8.

L'étape de clarification à pH acide selon l'invention est une étape de clarification par filtration en profondeur. Cette clarification est réalisée de manière à ne retenir que les plus grosses particules pouvant gêner le fonctionnement du lit fluidisé. De préférence, les filtres ayant un taux de rétention nominale de 15 à 100 µm, de préférence de 25 à 70 µm ou de 15 à 40 µm, sont utilisés. Dans un mode de réalisation, les filtres clarifiants de type SEITZ T5500 ayant un taux de rétention nominale de 25-70 µm sont utilisés à cette étape, et plus particulièrement le filtre T2600 dont le taux de rétention nominale est de 15-40 µm. Tout type de filtre, chargé ou non, permettant une clarification équivalente peut être utilisé. La clarification est avantageusement réalisée en présence de terres de filtration, permettant ainsi de réduire la surface de filtre à utiliser. Afin d'augmenter la fluidité de la solution à filtrer, la clarification est avantageusement réalisée entre 4 et 30 °C, plus particulièrement entre 10 et 25°C, par exemple entre 20 et 25°C. De façon avantageuse, le filtre utilisé dans l'étape de clarification selon l'invention a une capacité d'au moins 40 litres par m² soit 8 kg de précipité par m², plus précisément 55 litres par m² soit 11 kg de précipité par m².

A l'issue de la filtration en profondeur, le pH et l'osmolalité du surnageant de filtration sont avantageusement ajustés sans étape de dialyse ou d'ultrafiltration, en fonction des besoins de l'étape suivante du procédé.

Dans un mode de réalisation particulier, l'étape de chromatographie en lit fluidisé peut être une chromatographie de type « mode-mixte » qui comprend :
- la charge sur une colonne de chromatographie préalablement équilibrée avec un tampon à un pH compris entre 4,5 et 8, plus particulièrement entre 4,5 et 7,5 ; 4,5 et 7,0 ; 4,5 et 6,8 ; 4,5 et 6,5 ; 5,0 et 7,0 ; 5,0 et 6,5 ; 5,5 et 6,3 ; ou entre 5,7 et 6,3 ; de la solution ayant subi l'étape de clarification par filtration en profondeur préalablement ajustée au même pH, c'est-à-dire à un pH compris entre 4,5 et 8, plus particulièrement entre 4,5 et 7,5 ; 4,5 et 7,0 ; 4,5 et 6,8 ; 4,5 et 6,5 ; 5,0 et 7,0 ; 5,0 et 6,5 ; 5,5 et 6,3 ; ou entre 5,7 et 6,3,
- le lavage de la susdite colonne par une solution tampon jusqu'à élimination de toutes les protéines non adsorbées sur la colonne,
- l'élution des immunoglobulines polyvalentes adsorbées sur la colonne par un tampon d'élution ajusté à un pH compris entre 8 et 10, de préférence entre 8 et 9,8 ; 8,3 et 9,8 ; 8,5 et 9 ,8 ; 8,8 et 9,8 ; 9,0 et 9,8 ; 9,5 et 9,8, et
- la récupération de la solution enrichie en immunoglobulines polyvalentes humaines.

La chromatographie de type « mode-mixte » en lit fluidisé mise en oeuvre dans l'invention utilise, de préférence, des billes ayant des tailles différentes, ce qui permet de stabiliser le lit de chromatographie. Les billes mises en oeuvre dans la chromatographie de type « mode-mixte » en lit fluidisé selon l'invention ont une densité moyenne de 2,5 à 3,5 kg/L, un diamètre compris entre 20 et 400 µm et une capacité de fixation des immunoglobulines polyvalentes dans les conditions de l'invention pouvant avantageusement dépasser 70 g/L.

L'élution mise en oeuvre dans la chromatographie en lit fluidisé est par exemple une élution à pH basique et à faible force ionique afin de pouvoir enchaîner directement l'étape suivante du procédé, après avoir procédé à des ajustements simples en pH et en conductivité. Un tampon d'élution utilisable dans le cadre de l'invention peut par exemple contenir : Glycine de 5 à 500 mM, notamment 20mM, et NaCl de 5 à 500 mM, notamment 20 mM, à pH compris entre 7 et 10, notamment compris entre 9 et 10, notamment compris entre 9,5 et 10, et de façon plus intéressante entre pH 9,5 et 9,8.

Dans le cas où l'étape suivant la chromatographie en lit fluidisé n'est pas compatible avec des conditions de force ionique élevées, et en conséquence nécessiterait préalablement une dilution ou une dialyse importante de l'éluat obtenu à l'issue de l'étape de chromatographie en lit fluidisé, un tampon d'élution ayant un pH élevé mais une force ionique faible peut être utilisé. Un tampon d'élution utilisable dans le cadre de l'invention peut par exemple contenir : Glycine de 5 à 20 mM, notamment 10 mM, à un pH compris entre 9,5 et 10,5, notamment compris entre 9,8 et 10,5 et de façon plus intéressante entre pH 9,8 et 10,2.

Dans un mode de réalisation particulier, le procédé selon l'invention comprend en outre, après l'étape de chromatographie en lit fluidisé, une ou plusieurs des étapes suivantes :
(i) une étape d'inactivation virale ;
(ii) une étape de chromatographie d'échange d'anions de la solution obtenue à l'issue de l'étape précédente ayant pour but d'éliminer les composants chimiques utilisés lors de l'étape d'inactivation virale et de réduire la contamination en IgA et IgM ;
(iii) une étape d'élimination d'anticorps anti-A et anti-B de la solution obtenue à l'issue de l'étape précédente ;
(iv) une étape de filtration au travers de filtres nanométriques de porosité décroissante de 100 à 15 nm ;
(v) une étape de concentration par ultrafiltration de la solution issue de l'étape précédente associée à une étape de formulation ;
(vi) une étape de filtration stérilisante conventionnelle.

Le concentré d'immunoglobulines polyvalentes obtenu selon le procédé de l'invention a généralement subi au moins une étape d'élimination ou d'inactivation d'au moins un agent infectieux. Parmi les agents infectieux, on peut citer les virus et les ATNC (agents transmissibles non conventionnels) comme le prion. Une inactivation virale comprend souvent un traitement avec des produits chimiques, par exemple par solvant, et/ou détergent et/ou par la chaleur, par exemple par UVC, irradiation Gamma, et/ou pasteurisation. Une nanofiltration est aussi utile pour éliminer un agent infectieux, notamment les virus. De préférence, le procédé comprend au moins un traitement par solvant et détergent, et une nanofiltration. Le traitement par solvant et/ou détergent (appelé généralement traitement solvant/détergent) comprend notamment le traitement par le tri-n-butylphosphate (TnBP) et/ou un détergent qui est choisi parmi le Triton X-100, le Tween (de préférence Tween 80), le cholate de sodium et le 2-[4-(2,4,4-triméthylpentane-2-yl)phénoxy]éthanol (Octoxinol). La nanofiltration se réfère généralement à la filtration du concentré d'immunoglobulines polyvalentes à travers un filtre avec une taille de pores inférieure à 80 nm. Les filtres disponibles sont par exemple les filtres BioEx, Planova ™ 75 nm, Planova ™ 35 nm, Planova ™ 20 nm ou Planova™ 15 nm (Asahi corporation), Ultipor DV 50 or DV 20 (Pall corporation), Virosart CPV (Sartorius), Viresolve NFR ou NFP (Millipore). De préférence, une nanofiltration est réalisée avant l'étape (v) de concentration des immunoglobulines polyvalentes par ultrafiltration associée à une étape de formulation. Dans un mode de réalisation particulier, le concentré d'immunoglobulines polyvalentes purifié est filtré sur une séquence de filtres avec une taille de pores entre 15 et 50 nm, par exemple de 20 ou 35 nm.

La chromatographie d'échange d'anions mise en oeuvre dans le procédé selon l'invention est effectuée à pH alcalin et faible force ionique pour permettre la fixation des immunoglobulines polyvalentes. Cette étape est réalisée par exemple selon le procédé décrit dans le brevet EP 1 385 886 (Laboratoire Français du Fractionnement et des Biotechnologies, « Procédé de préparation de concentrés d'immunoglobulines humaines à usage thérapeutique »). La résine est équilibrée par tout tampon ayant un pouvoir tampon à faible concentration pour des pH compris entre 8,5 et 9,5, tel que les tampons Tris ou plus particulièrement glycine. La concentration en glycine peut être comprise entre 5 et 50 mM, par exemple entre 5 et 20 mM et plus particulièrement entre 8 et 10 mM. Le pH sera ajusté entre 8,5 et 9,5 et plus particulièrement entre 8,9 et 9,1.

De façon avantageuse, l'éluat de la chromatographie en lit fluidisé est dilué et ajusté en pH de manière à permettre la fixation des immunoglobulines polyvalentes sur le support chromatographique échangeur d'anions. Cette dilution est effectuée avec de l'eau de façon à amener la conductivité sous une valeur de 1500 µS/cm et plus préférablement sous 1100 µS/cm. La colonne est lavée dans le tampon d'équilibrage, puis les immunoglobulines polyvalentes sont éluées en tampon Na/Na₂ PO₄ 20 mM à pH 6,2. Enfin, la colonne est lavée avec une solution de NaCl à 150 mM.

L'élimination d'anticorps anti-A et anti-B dans la solution obtenue à partir de la chromatographie d'échange d'anions peut être effectuée selon la méthode décrite dans la demande de brevet WO 2007/077365 (Laboratoire Français du Fractionnement et des Biotechnologies, « Concentré d'immunoglobulines G (IgG) appauvri en anticorps anti-A et anti-B et en IgG polyréactives »). La solution obtenue à l'étape précédente est soumise à une étape d'élimination des anticorps anti-A et anti-B par chromatographie d'immunoaffinité par percolation dudit concentré d'immunoglobulines polyvalentes sur un mélange de supports dont les matrices sont greffées de groupes oligosaccharides antigéniquement similaires aux groupes sanguins A et B.

Dans un mode de réalisation particulier de l'invention, la solution initiale est du plasma sanguin ou une fraction de plasma enrichie en immunoglobulines par des méthodes bien connues de l'homme du métier, et notamment par fractionnement éthanolique et/ou par séparation chromatographique.

Dans un mode de réalisation particulier, la solution initiale est un précipité « I+II+III » ou un précipité « II+III » obtenu à partir de plasma sanguin fractionné à l'éthanol selon la méthode de Cohn ou de Kistler et Nistschmann (1962, Vox Sang. 7, 414), et remis en solution. Avantageusement, le précipité « I+II+III » ou le précipité « II+III » est remis en solution dans le cadre de l'invention dans de l'eau purifiée pour injection (ppi) ou dans une solution contenant des ions. La solution contenant des ions utilisée dans le cadre de l'invention peut être une solution comprenant du NaCl à une concentration inférieure ou égale à 20 mM, de préférence comprise entre 5 et 15 mM, et de manière préférée égale à 10 mM.

Dans un autre mode de réalisation, la remise en solution du précipité « I+II+III » ou du précipité « II+III » est réalisée dans des conditions permettant la précipitation du fibrinogène contaminant. La précipitation du fibrinogène contaminant peut alors être obtenue par traitement du précipité « I+II+III » ou du précipité « II+III » avec une solution précipitante spécifique du fibrinogène, par exemple une solution de CaCl₂ dont la concentration est inférieure ou égale à 20 mM, de préférence comprise entre 5 et 15 mM, et de manière préférée avec une solution de sels 10 mM. Dans un tel mode de réalisation, le fibrinogène reste sous forme précipitée alors que les immunoglobulines polyvalentes sont resolubilisées.

Un mode de réalisation particulier de l'invention concerne un procédé de préparation d'un concentré d'immunoglobulines polyvalentes humaines, comprenant :
(i) une étape d'élimination de contaminants protéiques par l'acide caprylique ;
(ii) une étape de clarification par filtration en profondeur ;
(iii) une étape de chromatographie en lit fluidisé, de type « mode-mixte » ;
(iv) une étape d'inactivation virale par traitement solvant/détergent ;
(v) une étape de chromatographie d'échange d'anions ;
(vi) une étape d'élimination des anticorps anti-A et anti-B ;
(vii) une filtration au travers de filtres nanométriques de porosité décroissante de 100 à 15 nm ;
(viii) une étape de concentration par ultrafiltration de la solution issue de l'étape précédente associée à une étape de formulation, puis une étape de filtration stérilisante conventionnelle.

Dans un mode de réalisation plus particulier, l'invention concerne un procédé de préparation d'un concentré d'immunoglobulines polyvalentes humaines, comprenant :
(i) le traitement d'une solution initiale de plasma sanguin ou d'une fraction de plasma enrichie en immunoglobulines par fractionnement à l'éthanol, pour obtenir un précipité « I+II+III » ou « II+III », selon la méthode de Cohn (déjà cité) ou de Kistler et Nitschmann (1962, Vox Sang. 7, 414) ;
(ii) la remise en solution du précipité « I+II+III » ou « II+III » avec de l'eau ppi, sous agitation, à 20°C (préférentiellement 4 à 15°C). Différents ions peuvent être apportés afin de faciliter la remise en solution des immunoglobulines polyvalentes précipitées, par exemple des cations tels que le Na⁺, des anions tels que le Cl⁻, par exemple du NaCl. La concentration du sel est inférieure ou égale à 20 mM, de préférence comprise entre 5 et 15 mM, et de manière préférée égale à 10 mM, comme par exemple du NaCl à une molarité de 10 mM ;
(iii) la précipitation à partir du précipité « I+II+III » ou « II+III » remis en solution, de contaminants protéiques par l'acide caprylique à une concentration de 0,5 à 1,5%, notamment de 0,8 à 1,2%, particulièrement de 0,9 à 1,1%, pour obtenir une solution plasmatique dépourvue en protéases, puis l'ajustement du pH entre 4,3 et 4,9, préférablement entre 4,4 et 4,8, et de préférence entre 4,6 et 4,8. Dans un mode de réalisation, l'ajustement du pH se fait avec du HCl ;
(iv) la filtration de la solution plasmatique dépourvue en protéases issue de l'étape (iii) à pH acide par un filtre en profondeur ayant la porosité la plus large possible permettant l'injection du filtrat sur une colonne de chromatographie utilisée en mode lit fluidisé ;
(v) un ajustement du pH et de l'osmolalité de la solution issue de l'étape (iv),
(vi) une chromatographie en lit fluidisé de la solution obtenue à l'issue de l'étape précédente, pour obtenir une solution sanguine enrichie en immunoglobulines polyvalentes humaines. De façon avantageuse, ladite chromatographie doit permettre une élution des immunoglobulines polyvalentes dans un tampon proche du tampon nécessaire aux étapes suivantes du procédé, afin de limiter les pertes d'immunoglobulines polyvalentes qui pourraient être dues à une étape de dialyse, d'ultrafiltration ou toute étape nécessaire à la reformulation de la solution ;
(vii) le traitement par solvant/détergent comprenant notamment le traitement par le tri-n-butylphosphate (TnBP) et/ou un détergent qui est choisi parmi le Triton X-100, le Tween (de préférence Tween 80), le cholate de sodium et le 2-[4-(2,4,4-triméthylpentane-2-yl)phénoxy]éthanol (Octoxinol), pour inactiver des virus dans la solution obtenue à l'issue de l'étape précédente ;
(viii) la chromatographie d'échange d'anions de la solution obtenue à l'issue de l'étape précédente, utilisant un gel de polysaccharide réticulé ou de polymère vinylique, greffé de groupements TMAE afin d'éliminer les solvants et détergents utilisés à l'étape précédente et de retirer des contaminants de type IgA et IgM ;
(ix) la chromatographie d'affinité telle que décrite dans la demande de brevet WO 2007/077365 (Laboratoire Français du Fractionnement et des Biotechnologies, « Concentré d'immunoglobulines G (IgG) appauvri en anticorps anti-A et anti-B et en IgG polyréactives ») pour éliminer les anticorps anti-A et anti-B de la solution obtenue à l'issue de l'étape précédente ;
(x) la nanofiltration de la solution obtenue à l'issue de l'étape précédente, utilisant un préfiltre de porosité 50 (par exemple sur un filtre PALL DV20) ou 35 nm (par exemple sur un filtre PLANOVA 35 N), suivie d'un filtre de porosité 20 (par exemple un filtre PLANOVA 20 N) ou 15 nm (par exemple un filtre PLANOVA 20 N ou BIOEX) pour éliminer des prions ou des virus résistants au traitement de l'étape (ix) ;
(xi) la concentration ou diafiltration de la solution obtenue à l'issue de l'étape précédente, pour obtenir une solution contenant au moins 50g/L d'immunoglobulines polyvalentes.

La solution obtenue à l'issue de l'étape (iii) d'élimination des contaminants par l'acide caprylique du procédé de l'invention possède une concentration en protéases conforme à la norme 2.6.15 de la Pharmacopée Européenne 6.7, Edition 01/2012 :0918, laquelle est au maximum de 35 UI/mL calculée par rapport à une dilution de la préparation à examiner contenant 30 g/L d'immunoglobulines.

L'invention a également pour objet un concentré d'immunoglobulines polyvalentes humaines obtenu par le procédé de l'invention caractérisé en ce que ledit concentré possède une activité anti-complémentaire inférieure à 30 %.

L'invention a également pour objet un procédé de préparation d'une composition pharmaceutique d'immunoglobulines polyvalentes humaines liquide, congelée ou lyophilisée, caractérisée en ce que :
a. l'addition au concentré d'immunoglobulines polyvalentes humaines, obtenu selon le procédé décrit dans l'invention, d'un ou plusieurs stabilisants pharmaceutiquement acceptables, et
b. optionnellement, la congélation ou la lyophilisation de la préparation pharmaceutique obtenue à l'étape précédente ;
de sorte que ladite préparation pharmaceutique soit sous forme liquide, congelée ou lyophilisée.

Les stabilisants pharmaceutiquement acceptables utilisés dans le cadre de l'invention sont par exemple ceux décrits dans les demandes de brevet FR 2 853 551, FR 2 961 107 et FR 2 962 650. La demande FR 2 853 551 décrit une formulation stabilisante comprenant un sucre alcoolique tel que le mannitol, de la glycine et un détergent non ionique. La concentration en mannitol est avantageusement comprise entre 30 g/l et 50 g/l, celle de la glycine entre 7 g/l et 10 g/l et celle du détergent non ionique entre 20 et 50 ppm. La demande FR 2 961 107 décrit une formulation stabilisante comprenant de la glycine et un détergent non ionique à un pH inférieur ou égal à 4,8. La concentration de glycine est au moins de 200 mM, de préférence de 250 mM ± 50 mM et celle du détergent non ionique est comprise entre 20 et 100 mg/l, de préférence 35 mg/l ± 15 mg/l, de préférence encore 50 mg/l. La demande FR 2 962 650 décrit l'ajout à une préparation d'immunoglobulines G humaines d'excipients choisis parmi les acides aminés, les sucres, les dérivés des sucres, les sels et les tensioactifs, lesdits tensioactifs étant ajoutés à une concentration inférieure à la concentration micellaire critique desdits tensioactifs.

L'invention a aussi pour objet l'utilisation d'une composition pharmaceutique d'immunoglobulines polyvalentes humaines liquide, congelée ou lyophilisée, en particulier une composition pharmaceutique d'immunoglobulines polyvalentes humaines liquide, congelée ou lyophilisée obtenue selon le procédé de l'invention, pour le traitement de pathologies, telles que les polyneuropathies démyélinisantes, la sclérose en plaques, le syndrome myasthénique de Lambert-Eaton, les dermatomyosites, pour le traitement substitutif dans le cas de déficits immunitaires primitifs tels que les agammaglobulinémies congénitales et les hypogammaglobulinémies congénitales, le déficit immunitaire commun variable, le déficit immunitaire combiné sévère, le syndrome de Wiskott Aldrich, le myélome ou la leucémie lymphoïde chronique avec hypogammaglobulinémie secondaire sévère et infections récurrentes, les infections récurrentes chez l'enfant infecté par le VIH , ou pour le traitement de déficits immunitaires primitifs avec hypogammaglobulinémie ou atteinte fonctionnelle de l'immunité humorale. La présente invention comprend également l'utilisation de la composition pharmaceutique d'immunoglobulines polyvalentes humaines liquide, congelée ou lyophilisée de l'invention pour le traitement de déficits immunitaires secondaires de l'immunité humorale, en particulier la leucémie lymphoïde chronique ou le myélome avec hypogammaglobulinémie et associés à des infections à répétition, l'allogreffe de cellules souches hématopoïétiques avec hypogammaglobulinémie associée à une infection, ainsi que le traitement immunomodulateur du purpura thrombopénique idiopathique (PTI) chez l'adulte et l'enfant en cas de risque hémorragique important ou avant un acte médical ou chirurgical pour corriger le taux de plaquettes, de la rétinochoroïdite de Birdshot, du syndrome de Guillain-Barré, de la neuropathie motrice multifocale (NMM), des polyradiculonévrites inflammatoires démyélinisantes chroniques (PIDC), et de la maladie de Kawasaki.

Les exemples suivants décrivent des modes de réalisation du procédé selon l'invention. Les exemples ci-dessous visent à éclairer l'objet de l'invention et illustrer des modes de réalisation avantageux, mais en aucun cas ne visent à restreindre la portée de l'invention.

### EXEMPLES

### EXEMPLE 1

### 1.1 Remise en solution du précipité « I+II+III »

On utilise comme matériel de départ le précipité « I+II+III » obtenu à partir de plasma sanguin fractionné à l'éthanol selon la méthode de Cohn ou de Kistler et Nistschmann (1962, Vox Sang. 7, 414). Ce précipité est remis en solution à raison de 56,7 g pour 228 ml d'eau déminéralisée ou proportions équivalentes. Agitation du mélange pendant 20 minutes à 15°C ± 5°C. Puis la température est remontée à 22°C ± 2°C et l'acide caprylique (1% poids/volume) est ajouté lentement dans le précipité « I+II+III » remis en solution. Le pH de la solution obtenue après l'addition d'acide caprylique est ajusté à 4,8 et la solution reste sous agitation pendant 60 minutes.

### 1.2 Filtration en profondeur

La solution résultante est clarifiée par une filtration en profondeur sur filtre SEITZ® T 2600 (Pall Corporation). Cette filtration retient à la fois les adjuvants de filtration présents dans le précipité « I+II+III » et le précipité protéique généré par l'addition d'acide caprylique.

Le pH de l'échantillon est également avantageusement étudié pour optimiser le rendement de filtration.

Le pH de la solution protéique est ajusté à 6,0 uniquement après la filtration. En effet, l'ajustement du pH de 4,8 à 6,0 génère un léger précipité (insolubilité des immunoglobulines polyvalentes) retenu par le filtre et donc une diminution du rendement de filtration en immunoglobulines polyvalentes.

Le volume de rinçage du filtre (eau purifiée apyrogène) est équivalent au volume de l'échantillon de départ afin d'optimiser le rendement final.

### 1.3 Chromatographie de type « mode-mixte » en lit fluidisé

Les conditions opératoires sont les suivantes :
- Tampon d'équilibrage et de lavage : 20 mM de Na/Na₂PO₄ à pH 6.
- Tampon d'élution : 20 mM Glycine/20 mM de NaCl à pH 9,8.

Le choix d'une élution par effet de pH dans un tampon Gly/NaCl permet par simple dilution (ajustement de la conductivité et du pH) d'obtenir un échantillon prêt à être injecté sur la colonne d'échange d'ions (TMAE Fractogel).

### 1.4 Traitement par solvant/détergent

L'éluat de la chromatographie de type « mode-mixte » en lit fluidisé selon l'exemple 1.3 subit un traitement d'inactivation virale par solvant/détergent comme décrit par Neurath et Horowitz (US 4,764,369).

Le mélange solvant/détergent 10 fois concentré contient 3 % de TnBP (tri(n-butyl)phosphate) et 10% d'Octoxinol. La concentration finale dans l'éluat est de 0,3 % de TnBP et 1 % d'Octoxinol.

Après 1 heure d'inactivation, l'éluat est ajusté à pH 9,0 et dilué en eau pour obtenir une conductivité inférieure à 1100 µS/cm.

### 1.5 Chromatographie d'échange d'ions

Le gel d'échange d'ions mis en oeuvre est le TMAE-Fractogel®, Merck.
- Tampon de pré-équilibrage : glycine 80 mM / NaCl 80 mM à pH 9.
- Tampon d'équilibrage : glycine 9 mM / NaCl 9 mM à pH 9.

Après la charge de l'échantillon, la colonne est lavée avec le tampon d'équilibrage. Puis, elle est éluée en tampon Na/Na₂ PO₄ 20 mM à pH 6,2.

### 1.6 Chromatographie d'affinité

Le gel d'affinité mis en oeuvre est l'Iso A/Iso B Hypercel, Pall Corporation.

L'éluat de la chromatographie TMAE est soumis à cette chromatographie d'affinité, afin d'éliminer les anticorps anti-A et anti-B. Les immunoglobulines polyvalentes d'intérêt traversent la colonne sans être retenues.
- Equilibrage du gel en eau déminéralisée
- Récupération de la fraction non-adsorbée sans lavage de la colonne.

### 1.7 Filtration

La solution protéique issue de la chromatographie d'affinité est filtrée sur un filtre en profondeur 90LA CUNO, 3M puis sur un filtre 0,2 µm. Les filtres sont rincés avec de l'eau et la solution de rinçage est incorporée au filtrat.

### 1.8 Nanofiltration

Le filtrat précédent, ajusté à pH 4,5, est préfiltré en ligne sur un filtre Fluorodyne II 0,1 µm, Pall Corporation et nanofiltré sur filtre DV50, Pall Corporation, puis sur un filtre Planova 20N, Asahi.

### 1.9 Formulation

Le produit est pré-concentré à environ 80 g/l par ultrafiltration sur une cassette de seuil de coupure 30 kDa, puis diafiltré à volume constant jusqu'à ce que la conductivité soit < 600 µS/cm. Le produit est alors formulé et ajusté à 50 g/l de protéines.

### EXEMPLE 2: Obtention d'un concentré d'immunoglobulines polyvalentes par la mise en oeuvre du procédé de l'invention à l'échelle pilote

### 2.1 Remise en solution du précipité « I+II+III »

On utilise comme matériel de départ le précipité « I+II+III » obtenu à partir de plasma sanguin fractionné à l'éthanol selon la méthode de Kistler et Nistschmann (1962, Vox Sang. 7, 414). Ce précipité est remis en solution à raison de 3 kg pour 12 L d'eau déminéralisée. Agitation du mélange pendant 20 minutes minimum à 10°C ± 3°C. Puis la température est remontée à 22°C ± 2°C et l'acide caprylique (1 % poids/volume) est ajouté lentement dans le précipité « I+II+II » remis en solution. Le pH de la solution obtenue après l'addition d'acide caprylique est ajusté à 4,8 et la solution reste en agitation pendant 60 minutes.

### 2.2 Filtration en profondeur

La solution résultante est clarifiée par une filtration en profondeur sur filtre SEITZ® T 2600 (Pall Corporation). Cette filtration retient à la fois les adjuvants de filtration présents dans le précipité « I+II+III » et le précipité protéique généré par l'addition d'acide caprylique. Pour cet essai, la surface utilisée est de 8,7 kg de précipité / m² de média filtrant.

Le volume de rinçage du filtre (eau purifiée apyrogène) est équivalent au volume de l'échantillon de départ soit 15 L.

### 2.3 Chromatographie de type « mode-mixte » en lit fluidisé

Le gel de chromatographie mis en oeuvre est le gel Rhobust IGIV, Upfront. 4,3 L de gel dans une colonne de diamètre 10 cm. Les conditions opératoires sont les suivantes :
- Tampon d'équilibrage et de lavage : 20 mM de Na/Na₂PO₄ à pH 6.
- Tampon d'élution : 20 mM Glycine/20 mM de NaCl à pH 9,8.

### 2.4 Traitement par solvant/détergent

L'éluat de la chromatographie de type « mode-mixte » en lit fluidisé subit un traitement d'inactivation virale par solvant/détergent comme décrit par Neurath et Horowitz (US 4, 764, 369).

Le mélange solvant/détergent 10 fois concentré contient 3 % de TnBP (tri-n-butylphosphate) et 10 % d'Octoxinol. La concentration finale dans l'éluat est de 0,3 % de TnBP et 1 % d'Octoxinol.

Après 1 heure minimum d'inactivation, l'éluat est ajusté à pH 9,0 et dilué avec de l'eau pour obtenir une conductivité inférieure à 1100 µS/cm.

### 2.5 Chromatographie d'échange d'ions

Le gel d'échange d'ions mis en oeuvre est le EMD-TMAE Fractogel®, Merck.

4 L de gel dans une colonne de diamètre 12,7 cm.
- Tampon de pré-équilibrage : glycine 80 mM / NaCl 80 mM à pH 9.
- Tampon d'équilibrage : glycine 9 mM / NaCl 9 mM à pH 9.

Après la charge de l'échantillon, la colonne est lavée dans le tampon d'équilibrage. Puis, elle est éluée en tampon Na/Na₂ PO₄ 20 mM à pH 6,2.

### 2.6 Chromatographie d'affinité

Le gel d'affinité mis en oeuvre est l'Iso A/Iso B Hypercel, Pall Corporation. 154 mL de gel dans une colonne de diamètre 7 cm.

L'éluat de la chromatographie TMAE est soumis à cette chromatographie d'affinité, afin d'éliminer les anticorps anti-A et anti-B. Les immunoglobulines polyvalentes d'intérêt traversent la colonne sans être retenues.
- Equilibrage du gel en eau déminéralisée
- Récupération de la fraction non-adsorbée sans lavage de la colonne.

### 2.7 Filtration

La solution protéique issue de la chromatographie d'affinité Iso A/Iso B Hypercel est filtrée sur un filtre en profondeur 90LA CUNO, 3M puis sur un filtre 0,2 µm. Le rinçage des filtres en eau est incorporé au filtrat.

### 2.8 Nanofiltration

Le filtrat précédent ajusté à pH 4,5 est préfiltré en ligne sur un filtre Fluorodyne II 0,1 µm, Pall Corporation et nanofiltré sur filtre DV50, Pall Corporation, puis sur un filtre Planova 20N, Asahi.

### 2.9 Formulation

Le produit est pré-concentré à environ 80 g/l par ultrafiltration sur une cassette de seuil de coupure 30 kDa, puis diafiltré à volume constant jusqu'à ce que la conductivité soit < 600 µS/cm. Le produit est alors formulé et ajusté à 50 g/l de protéines.

### EXEMPLE 3 : Rendement du procédé

Trois lots ont été réalisés avec trois précipités « I+II+III » différents selon le procédé selon l'invention décrit dans l'exemple 1 et précisé dans l'exemple 3. L'élimination des protéases est obtenue avec 1% d'acide caprylique. La filtration est effectuée en filtre-presse sur plaques SEITZ T2600.

Un lot témoin est traité selon le procédé de référence, tel que décrit dans la demande de brevet EP 1 385 886.

Les résultats de rendement du nouveau procédé sont montrés dans le tableau ci-dessous :

### Rendements cumulés en g/L de protéines ou d'Ig de plasma au stade concentré formulé

| | Lot selon l'invention | Lot témoin traité selon le procédé tel que décrit dans EP 1 385 886 |
|---|---|---|
| | 10AXTO1064 015 (Lot 1) | 10AXTO1064 038 |
| Rendement en g de protéines par litre de plasma traité. | 5,22 | 4,67 |
| Rendement en g d'Ig polyvalentes par litre de plasma traité | 4,75 | 4,49 |

Le procédé selon l'invention permet d'augmenter le rendement en g d'immunoglobulines polyvalentes par litre de plasma traité, par rapport au procédé de référence (EP 1 385 886).

Le gain dans l'exemple ci-dessus est de 0,26 g d'IgG/l de plasma, entre le lot selon l'invention et le lot témoin réalisé à partir du même précipité.

### EXEMPLE 4 : Activité anti-complémentaire

Trois lots exploratoires ont été réalisés selon le procédé de l'invention à la taille pilote à partir de trois précipités « I+II+II » différents. Ces lots ont été comparés à un lot réalisé à la même échelle (taille pilote), mais selon le procédé industriel de production des immunoglobulines en intraveineuse tel que décrit dans le brevet EP 1 385 886, et à trois lots industriels préparés selon le procédé décrit dans la demande EP 1 385 886 faits à partir des mêmes précipités.

Un lot fabriqué à la taille pilote correspond à un lot dont la taille représente au moins 10% du lot industriel.

Les lots selon l'invention présentent une activité anti-complémentaire (AAC) mesurée selon le test de la Pharmacopée Européenne 7.5, édition 01/2012 :0918, paragraphe 2.6.17, toujours inférieure à celles des lots réalisés selon le procédé décrit dans la demande EP 1 385 886. Par ailleurs, la progression observée en 18 mois sur ce critère est moindre pour les lots faits selon l'invention.

Les résultats d'activité anti-complémentaire (AAC) sont montrés dans le tableau ci-dessous :

| | T = 0 | T = +18 mois | Progression (%) |
|---|---|---|---|
| Lots exploratoires fabriqués à la taille pilote préparés selon le procédé de l'invention : | AAC % | AAC % | |
| Lot 1* | 28 | 31 | 11% |
| Lot 2** | 27 | 31 | 15% |
| Lot 3*** | 27 | 31 | 15% |
| Lot fabriqué à la taille pilote* préparé selon le procédé décrit dans la demande EP 1 385 886 : | 33 | 40 | 21% |
| Lots industriels préparés selon le procédé décrit dans la demande EP 1 385 886 : | | | |
| Lot industriel 1 * | 36 | 38 | 6% |
| Lot industriel 2** | 32 | 39 | 22% |
| Lot industriel 3*** | 32 | 37 | 16% |

| | | | |
|---|---|---|---|
| *, **, *** lots réalisés avec les mêmes précipités | | | |

Le procédé de l'invention permet donc d'obtenir un concentré d'immunoglobulines polyvalentes dont l'activité anti-complémentaire est inférieure à 30%.

### EXEMPLE 5 : Pureté des immunoglobulines produites suivant le procédé de l'invention.

Les données analytiques de pureté pour les lots pilotes décrits dans l'exemple 2 sont résumées dans le tableau ci-dessous.

| Analyses | Méthode de mesure | Lot Pilote N°1 | | Lot Pilote N°2 | | Lot Pilote N°3 | | Moyenne des lots industriels obtenus selon le procédé décrit dans la demande EP 1 385 886 | |
|---|---|---|---|---|---|---|---|---|---|
| pH | Ph.Eur (2.2.35) | 5,0 | | 4,8 | | 4,8 | | 4,9 | |
| Osmolalité mosmol/kg | Ph.Eur (2.2.29) | 297 | | 294 | | 299 | | 297 | |
| DTM Polymères (%) | Ph.Eur (2.2.29) | <0,4 | | <0,4 | | <0,4 | | 0,5 | |
| Dimères (%) | Ph.Eur (2.2.29) | 2,7 | | 3,7 | | 2,4 | | ND | |
| Monomères (%) | Ph.Eur (2.2.29) | 96,3 | | 95,3 | | 96,4 | | 98,7 * | |
| Fragments (%) | Ph.Eur (2.2.29) | 1 | | 1,1 | | 1,1 | | 1 | |
| | | | | | | | | | |
| Protéines totales (g/l) | Ph.Eur (2.5.33) | 51 | | 49 | | 49 | | 50 | |
| Taux de protéines (%) | Ph.Eur (2.5.33) | 98 | | 98 | | 98 | | | |

| | | | % protéines | | % protéines | | % protéines | | % protéines |
|---|---|---|---|---|---|---|---|---|---|
| IgG g/L | Néphélémétrie ** | 46,4 | 91 | 46 | 95 | 47,6 | 96 | 45,7 | 92 |
| IgG1 g/L | Néphélémétrie ** | 29,5 | 58 | 27,1 | 56 | 26,7 | 54 | 28 | 56 |
| IgG2 g/L | Néphélémétrie ** | 18 | 35 | 17,8 | 37 | 16,7 | 34 | 16,1 | 32 |
| IgG3 g/L | Néphélémétrie ** | 1,4 | 3 | 1,2 | 2 | 1,3 | 3 | 1,1 | 2 |
| IgG4 g/L | Néphélémétrie ** | 1,1 | 2 | 1 | 2 | 1 | 2 | 0,9 | 2 |
| IgGA g/L | ELISA *** | 7, 4 | 0,01 | 7,1 | 0,01 | 6 | 0,01 | 8,6 | 0,02 |
| IgGE g/L | Néphélémétrie ** | <0,8 | ND | <0,8 | ND | <0,8 | ND | <0,8 | ND |
| IgGM g/L | Néphélémétrie ** | 0,23 | | 0,2 | | 0,18 | | 0,16 | |
| Anticorps anti-Hbs Ag (UI/mL) | ELISA *** | 5,1 | | 3,5 | | 3,3 | | 4,6 | |
| Albumine mg/l | Néphélémétrie ** | <2,2 | | <2,2 | | <2,2 | | <2,2 | |
| Tranferrine mg/l | Néphélémétrie ** | <2,1 | | <2,1 | | <2,1 | | <2,1 | |
| Activateur de la prékallikréine | Ph.Eur (2.6.15) | <1 | | <1 | | <1 | | <2 | |
| Hémagglutinines anti-A | Ph.Eur (2.6.20) | 4 | | 16 | | 8 | | 8 | |
| Hémaglutinines anti-B | Ph.Eur (2.6.20) | 2 | | 4 | | 8 | | 4 | |
| Activité anti-complémentaire % | Ph.Eur (2.6.17) | 28 | | 27 | | 27 | | 33 | |

| | | | | | | | | | |
|---|---|---|---|---|---|---|---|---|---|
| Ph.Eur: Pharmacopée Européenne 6ième édition * Monomères + dimères. ** Pressac M, Later R. «Dosages sériques d'IgG, IgA, IgM, transferrine et haptoglobine. II Précision analytique et comparaison des résultats fournis par différents analyseurs ». Ann Biol Clin 1995 ; 53 : 273-81. *** R. A. Goldsby, T. J. Kindt, B. A. Osborne et J. Kuby, « Enzyme-Linked Immunosorbent Assay », in Immunology, 5e édition, pages 148-150, W. H. Freeman, New York, 2003 | | | | | | | | | |

La somme « monomères + dimères », la répartition des sous-classes, les taux de contaminants et l'activité anti-complémentaire sont équivalentes pour les immunoglobulines issues du procédé de l'invention ou du procédé de référence selon le brevet EP 1 385 886. Ces caractéristiques satisfont les critères de la Pharmacopée européenne.

### EXEMPLE 6 : Stabilité des immunoglobulines produites suivant le procédé de l'invention

Les produits formulés issu des lots pilotes décrits dans l'exemple 2 ont été conservés à 4°C et des aliquots des lots industriels réalisés avec les mêmes précipités de départ ont été conservés au même stade dans les mêmes conditions. Des prélèvements effectués tous les six mois ont permis d'observer sur trois critères les différences entre les lots obtenus selon l'invention (lots pilotes 1, 2 et 3) et les lots obtenus selon le procédé industriel de référence EP 1 385 886 (lots industriels 1, 2 et 3). Les trois critères mesurés sont les suivants :
- la mesure de la concentration en immunoglobulines G (IgG) et celle de la concentration en immunoglobulines anti-Hbs selon la méthode décrite dans la Pharmacopée européenne 6.7 (Pharmeuropa, volume 21, numéro 2, avril 2009), paragraphe 2.7.1, et
- la détermination de la taille moléculaire (polymères, dimères, monomères et fragments) selon la méthode décrite dans la Pharmacopée européenne 6.7 (Pharmeuropa, volume 21, numéro 2, avril 2009), paragraphe 2.2.29.

Le tableau ci-dessous rend compte de ces différences :

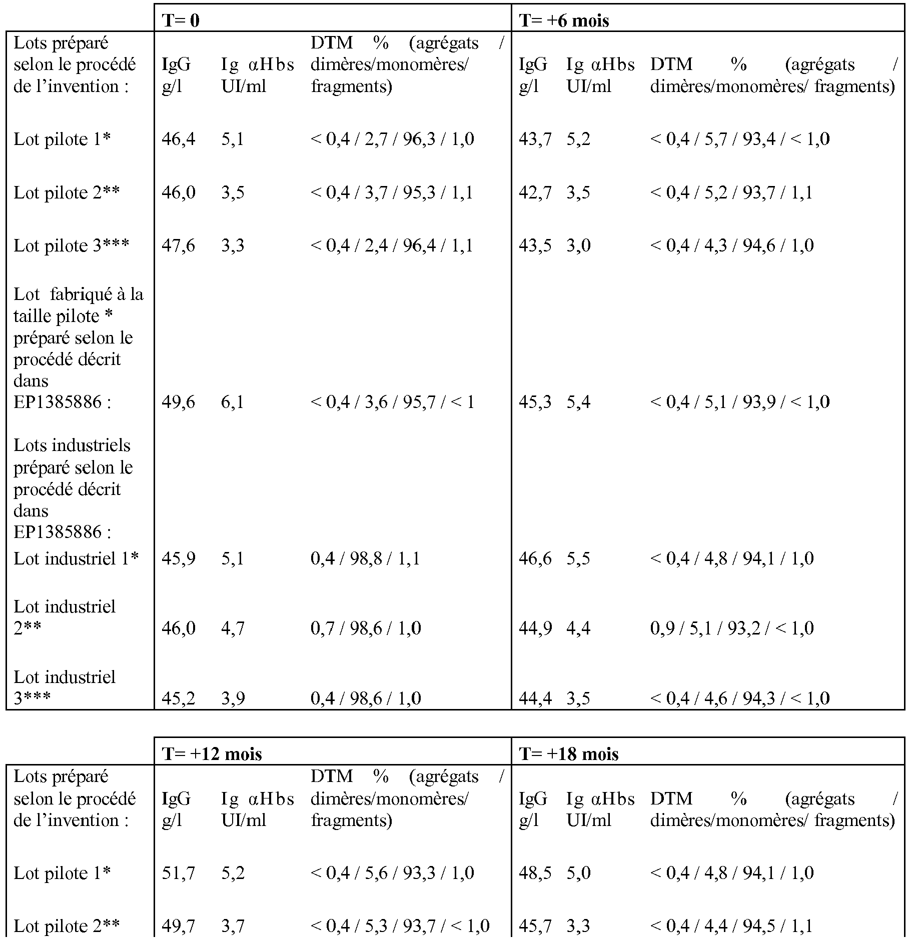

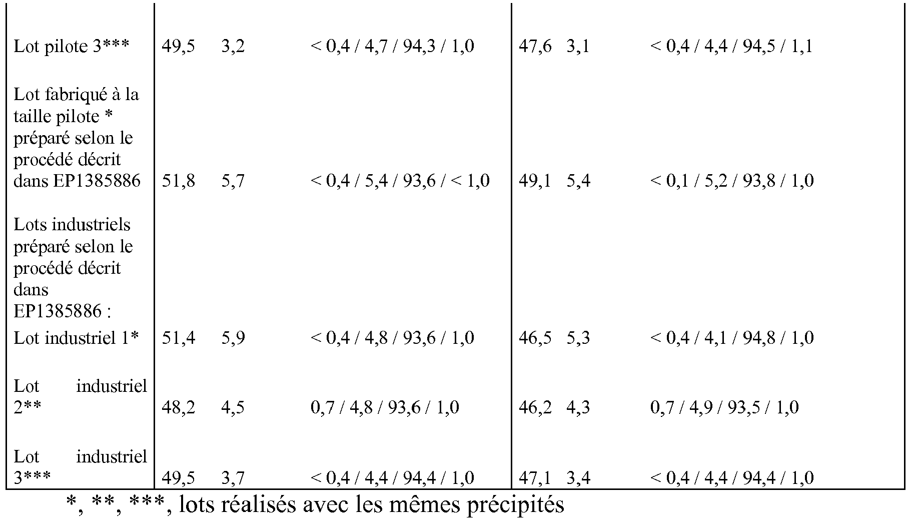

L'analyse des données montre :
- une stabilité de la concentration en IgG sur 18 mois et une homogénéité des 7 lots,
- une stabilité de la concentration des Ig anti-Hbs sur 18 mois : chaque lot étant spécifique sur ce critère,
- une somme « monomères + dimères » constante et supérieure aux 85% requis par la pharmacopée, et cela pour chaque lot sur 18 mois.

### EXEMPLE 7: Procédé comprenant une étape de précipitation caprylique, de clarification et une étape de chromatographie de type « mode-mixte » en lit fluidisé.

Le traitement à l'acide caprylique a pour but l'élimination par précipitation (les immunoglobulines restant en solution) d'une partie des protéines contaminantes issues du plasma et tout particulièrement des protéases. En fonction du pourcentage d'acide caprylique utilisé et de la méthode de séparation du surnageant et du précipité, une partie plus ou moins importante des immunoglobulines peut être perdue. Des conditions expérimentales ont été testées de manière à avoir la clarification la plus grossière possible compatible avec la chromatographie en lit fluidisé et générant la plus faible perte en immunoglobulines. Pour cela, des filtres de porosité croissante ont été comparés sans précipitation caprylique dans un premier temps, puis confirmés par la suite avec traitement à l'acide caprylique à différentes concentrations.

**Essais sur filtre de diamètre 90 soit 50 cm² (avec rinçage), pas de précipitation à l'acide caprylique**

| N° essai | 440 077 | 440 078 | 440 079 |
|---|---|---|---|
| Type de filtre | Pall Supradur50P | Pall/Seitz T 5500 | Pall/Seitz T 2600 |
| Porosité | 4-8 µm | 25-70 µm | 15-40 µm |
| pH | 4,8 | 4,8 | 4,8 |
| Volume passé ml | 150 | 150 | 150 |
| Rinçage passé | 0 | 75 | 75 |
| Volume récupéré ml | 9 (eau) | 208 | 216 |
| IgG départ g/l | 14,35 | 15,05 | 15,05 |
| IgG filtrat g/l | ND* | 10,5 | 10,2 |
| Rendement % | ND* | 97 | 98 |
| Volume/ Surface l/m² | ND* | 30 | 30 |

| | | | |
|---|---|---|---|
| * colmatage du filtre, pas de dosage effectué | | | |

Ces résultats montrent qu'une porosité minimale de l'ordre de 10-20 µm doit être mise en oeuvre afin de limiter la perte en IgG pour cause de colmatage. Le Filtre T2600 a été sélectionné par rapport au filtre T5500 pour sa porosité plus faible à un rendement en IgG équivalent.

Le pourcentage d'acide caprylique ajouté ayant un impact sur la précipitation des protéases et sur la filtrabilité de la solution, une gamme d'acide caprylique allant de 0,5 à 1% sans ajout d'adjuvent de filtration, a été testée. Les résultats obtenus sont comparés à ceux résultants d'une clarification témoin par simple centrifugation.

### Essai - Remise en suspension (RES) 1% acide caprylique - filtre de diamètre 90 soit 50 cm², charge 210 ml

| | | | RES pH 4,8(1) | RES Clarifiée (2) |
|---|---|---|---|---|
| Essai A | 1% Filtration SEITZ 2600 | IgG g/l | 13,7 | 9,7 |
| | | Rendement (Rdt) % | 100 | 76(3) |
| | | Protéases mDO/ml | 570 | 13 |
| Essai B | 0,5% Filtration SEITZ 2600 | IgG g/l | 15 | 9,4 |
| | | Rdt % | 100 | 51 (3) |
| | | Protéases mDO/ml | NR | NR |
| Essai C | 0,5% Centrifugation | IgG g/l | 14,1 | 12,4 |
| | | Rdt % | 100 | 72 |
| | | Protéases mDO/ml | 967 | 250 |

| | | | | |
|---|---|---|---|---|
| (1) : Précipité remis en solution ajustée à pH4,8 avant clarification (2) : Précipité remis en solution ajusté à pH et clarifié. (3) : Rinçages des filtres avec un volume correspondant à la moitié du produit à clarifier. | | | | |

Le témoin par centrifugation montre qu'une partie des immunoglobulines est perdue dans le précipité, si celui-ci n'est pas rincé, cette technique n'a pas été retenue. La condition la plus intéressante testée est la concentration de 1% en acide caprylique : la contamination en protéases est faible et la perte en immunoglobulines est inférieure à 30%. Une concentration de 1 % d'acide caprylique apparait donc être le meilleur compromis entre la filtrabilité, le rendement et l'élimination des protéases.

Afin d'augmenter le rendement, l'essai ci-dessous a été réalisé avec une charge plus faible (180 ml au lieu de 210) et en augmentant le volume de lavage du précipité avec le tampon de rinçage tel que décrit dans le tableau ci-dessous.

### Essai 09AXTO440 126 - RES 1% acide caprylique. - filtre Ø90 soit 50 cm², charge 180 ml.

| Etapes | Volume (ml) | IgG (g/l) | Quantité (mg) | Rendement (Rdt) % |
|---|---|---|---|---|
| RES pH 4,8 | 210 | 13,65 | 2866,5 | 100 |
| RES filtrée | 180 | 11,2 | 2016,0 | 70,3 |
| Rinçage 1 | 30 | 3,71 | 111,3 | 3,9 |
| Rinçage 2 | 30 | 2,26 | 67,8 | 2,4 |
| Rinçage 3 | 30 | 2,28 | 68,4 | 2,4 |
| Rinçage 4 | 30 | 1,71 | 51,3 | 1,8 |
| Rinçage 5 | 30 | 1,16 | 34,8 | 1,2 |
| Rinçage 6 | 30 | 0,91 | 27,3 | 1 |
| Rinçage 7 | 30 | 0,91 | 27,3 | 1 |
| Pool filtré | 385 | 6,95 | 2675,8 | 93,3 |

En diminuant le volume de chargement sur le filtre et en rinçant le précipité avec un volume de lavage équivalent au volume de produit à clarifier (180 ml), aucun colmatage n'est observé et la totalité des immunoglobulines a été récupérée. La totalité des immunoglobulines est disponible pour l'étape de chromatographie d'affinité qui suit dans le procédé de purification.

### EXEMPLE 8 : Amélioration de la remise en solution des IgG

Lors de la fabrication des lots taille pilote selon le nouveau procédé tel que décrit dans l'exemple 2, la comparaison avec le lot préparé selon le procédé décrit dans la demande de brevet EP 1 385 886 à la même échelle (10AXTO1064015) fait apparaitre que la remise en solution du précipité « I+II+III » peut avantageusement être améliorée. En effet, un différentiel d'au moins 1 g/l d'IgG est observé à cette étape (les volumes étant équivalents) comme le prouve le tableau ci-dessous.

| **Remise en solution non filtrée** | | | |
|---|---|---|---|
| Lot taille pilote préparé selon le procédé décrit dans la demande de brevet EP 1 385 886 (g/l) * | Lots taille pilote préparé selon le procédé de l'invention (g/l) | | |
| | Lot 1* | Lot 2 | Lot 3 |
| 12,8 | 11,6 | 10,3 | 11,7 |

| | | | |
|---|---|---|---|
| * lots réalisés avec le même précipité | | | |

Des essais complémentaires ont montré que la remise en solution des immunoglobulines présentes dans le précipité peut être avantageusement effectuée avec une solution contenant des ions plutôt qu'avec de l'eau purifiée. En particulier, en utilisant une solution de NaCl 10 mM.

Par ailleurs, la précipitation des contaminants protéiques par l'acide caprylique peut être également améliorée à des pH inférieurs à 4,8. En particulier, un pH de 4,6 permet cette amélioration.

Un lot à la taille pilote (« nouveau lot ») a été réalisé en tenant compte de ces améliorations et comparé au stade « Eluat chromatographie Rhobust » avec les trois lots (lots 1, 2 et 3) déjà réalisés selon l'invention.

Le tableau ci-dessous rend compte des différences :

### Rendement du nouveau procédé au stade « Eluat de la chromatographie en lit fluidisé »

| | Nouveau lot | Lot 1 | Lot 2 | Lot 3 |
|---|---|---|---|---|
| pH de RES | 4,6 | 4,8 | 4,8 | 4,8 |
| g IgG/l plasma | 6,00 | 5,96 | 5,62 | 5,77 |
| IgG/Protéines | 0,910 | 0,820 | 0,812 | 0,893 |

Le nouveau lot, réalisé avec une remise en solution avec une solution ayant une concentration en NaCl de 10 mM, traité par l'acide caprylique et ajusté à un pH égal à 4,6 présente :
- un meilleur rendement en immunoglobulines par litre de plasma mis en oeuvre,
- une meilleure pureté en regard du ratio immunoglobulines/protéines totales.

### EXEMPLE 9 : Application de l'invention au précipité « II+III »

Le précipité « II+III » est une alternative au précipité « I+II+III » comme source de matière première pour la purification d'IgG polyclonales.

A ce titre, deux lots selon l'invention ont été réalisés à partir de précipités « II+III » différents. Les résultats des premières évaluations obtenus à partir de ces deux matières premières ont été comparés au stade « Eluat de la chromatographie en lit fluidisé ».

Le tableau ci-dessous rend compte de ces résultats :

| | Lot « I+II+III » | Lot « II+III » | Lot « II+III » |
|---|---|---|---|
| pH de RES | 4,6 | 4,6 | 4,6 |
| g IgG/L de plasma | 6,00 | 5,17 | 5,1 |
| Rdt de purification % | 98 | 88 | 95 |
| IgG/Protéines | 0,910 | 0,837 | 0,859 |

Ces résultats montrent que la réalisation des premières étapes du procédé selon l'invention se déroule de façon équivalente avec les deux types de précipité :
- en rendement d'extraction : les rendements cumulés des étapes de clarification et de chromatographie d'affinité Rhobust sont comparables même si l'un des lots est un peu en retrait.

Toutefois, les précipités « II+III » qui ont subi une étape de fractionnement éthanolique supplémentaire par rapport au précipité « I+II+III » présentent un rendement en g d'IgG/L de plasma de 15% inférieur à celui obtenu avec le précipité « I+II+III ». Ce déficit est lié à la perte d'une partie des IgG au cours de l'étape de fractionnement éthanolique supplémentaire.
- en pureté : les ratios IgG/protéines totales obtenus avec les précipités « II+III » remis en solution, traités par l'acide caprylique et ajustés à un pH de 4,6 sont comparables à ceux obtenus avec les précipités « I+II+III » remis en solution, traités par l'acide caprylique et ajustés à un pH de 4,8.

## Revendications

1. Procédé de préparation d'un concentré d'immunoglobulines polyvalentes humaines à partir d'une solution initiale de plasma sanguin ou de fraction de plasma enrichie en immunoglobulines, **caractérisé en ce qu'**il comprend :
(a) une étape d'élimination des contaminants protéiques par l'acide caprylique pour obtenir une solution dépourvue en protéases, dans laquelle la concentration en acide caprylique utilisée va de 0,5 à 1,5%, exprimée en gramme d'acide caprylique par volume de solution à traiter,
(b) une étape de clarification à pH acide par filtration en profondeur,
(c) suivie d'une étape de chromatographie en lit fluidisé de la solution dépourvue en protéases,
ledit procédé permettant d'obtenir un concentré d'immunoglobulines polyvalentes humaines avec un rendement supérieur à 4,5 g d'immunoglobulines par litre de plasma sanguin mis en oeuvre.

2. Procédé selon la revendication 1, **caractérisé en ce que** la concentration d'acide caprylique utilisée va de 0,8 à 1,2 %, exprimée en gramme d'acide caprylique par volume de solution à traiter.

3. Procédé selon la revendication 2, **caractérisé en ce que** la concentration d'acide caprylique utilisée va de 0,9 à 1,1 %, exprimé en gramme d'acide caprylique par volume de solution à traiter.

4. Procédé selon l'une des revendications 1 à 3, **caractérisé en ce que** l'étape d'élimination des contaminants protéiques par l'acide caprylique (a) se déroule à un pH compris entre 4,3 et 4,9, de préférence compris entre 4,6 et 4,8.

5. Procédé selon l'une quelconque des revendications 1 à 4, **caractérisé en ce que** l'étape de chromatographie en lit fluidisé de la solution dépourvue en protéases (c) est effectuée avec un support chromatographique de type échange d'ions, de type affinité, de type « pseudo-affinité » ou de type « mode-mixte ».

6. Procédé selon la revendication 5, dans lequel l'étape de chromatographie en lit fluidisé de type « mode-mixte » comprend :
- la charge sur une colonne de chromatographie préalablement équilibrée avec un tampon à un pH compris entre 4,5 et 8 de la solution ayant subi l'étape de clarification par filtration en profondeur préalablement ajustée au même pH,
- le lavage de la susdite colonne par une solution tampon jusqu'à élimination de toutes les protéines non adsorbées sur la colonne,
- l'élution des immunoglobulines polyvalentes adsorbées sur la colonne par un tampon d'élution ajusté à pH compris entre 8 et 10, et
- la récupération de la solution enrichie en immunoglobulines polyvalentes humaines.

7. Procédé selon l'une quelconque des revendications 1 à 6, comprenant en outre, après l'étape (c), une ou plusieurs des étapes suivantes :
(i) une étape d'inactivation virale,
(ii) une étape de chromatographie d'échange d'anions de la solution obtenue à l'issue de l'étape précédente,
(iii) une étape d'élimination d'anticorps anti-A et anti-B de la solution obtenue à l'issue de l'étape précédente,
(iv) une étape de filtration au travers de filtres nanométriques de porosité décroissante de 100 à 15 nm,
(v) une étape de concentration par ultrafiltration de la solution issue de l'étape précédente associée à une étape de formulation,
(vi) puis une étape de filtration stérilisante conventionnelle.

8. Procédé selon l'une quelconque des revendications 1 à 7, dans lequel la solution initiale est une fraction de plasma enrichie en immunoglobulines par fractionnement éthanolique et/ou par séparation chromatographique.

9. Procédé selon la revendication 8, dans lequel la solution initiale est un précipité « I+II+III » ou un précipité « II+III » obtenu à partir de plasma sanguin fractionné à l'éthanol, et remis en solution.

10. Procédé selon la revendication 9, dans lequel le précipité « I+II+III » ou le précipité « II+III » est remis en solution dans de l'eau purifiée pour injection ou dans une solution contenant des ions.

11. Procédé selon la revendication 10, dans lequel la solution contenant des ions est une solution comprenant du NaCl à une concentration inférieure ou égale à 20 mM, de préférence comprise entre 5 et 15 mM, et de manière préférée égale à 10 mM.

12. Procédé selon l'une quelconque des revendications 8 à 11, dans lequel le précipité « I+II+III » ou le précipité « II+III » est traité par une solution de CaCl₂ dont la concentration est inférieure ou égale à 20 mM, de préférence comprise entre 5 et 15 mM, et de manière préférée avec une solution de CaCl₂ 10mM.

13. Procédé selon l'une quelconque des revendications 1 à 12, comprenant :
(i) une étape d'élimination de contaminants protéiques par l'acide caprylique,
(ii) une étape de clarification par filtration en profondeur,
(iii) une étape de chromatographie en lit fluidisé de type « mode-mixte »,
(iv) une étape d'inactivation virale par traitement solvant/détergent,
(v) une étape de chromatographie d'échange d'anions,
(vi) une étape d'élimination des anticorps anti-A et anti-B,
(vii) une filtration au travers de filtres nanométriques de porosité décroissante de 100 à 15 nm,
(viii) une étape de concentration par ultrafiltration de la solution issue de l'étape précédente associée à une étape de formulation, puis une étape de filtration stérilisante conventionnelle.

14. Procédé selon l'une quelconque des revendications 1 à 13, comprenant en outre les étapes suivantes :
(a) l'addition au concentré d'immunoglobulines polyvalentes humaines obtenu selon l'une quelconque des revendications 1 à 13, d'un ou plusieurs stabilisants pharmaceutiquement acceptables, et
(b) optionnellement, la congélation ou la lyophilisation de la préparation pharmaceutique obtenue à l'étape précédente ;
de sorte que ladite préparation pharmaceutique soit sous forme liquide, congelée ou lyophilisée.

15. Concentré d'immunoglobulines polyvalentes humaines obtenu selon le procédé de l'une quelconque des revendications 1 à 14, **caractérisé en ce que** ledit concentré possède une activité anti-complémentaire inférieure à 30%.

16. Composition pharmaceutique d'immunoglobulines polyvalentes humaines liquide, congelée ou lyophilisée obtenue selon le procédé de l'une quelconque des revendications 1 à 14 pour une utilisation dans le traitement de pathologies comprises dans le groupe consistant en les polyneuropathies démyélinisantes, la sclérose en plaques, le syndrome myasthénique de Lambert-Eaton, les dermatomyosites, un traitement substitutif dans le cas de déficits immunitaires primitifs tels que les agammaglobulinémies congénitales et les hypogammaglobulinémies congénitales, le déficit immunitaire commun variable, le déficit immunitaire combiné sévère, le syndrome de Wiskott Aldrich, le myélome ou la leucémie lymphoïde chronique avec hypogammaglobulinémie secondaire sévère et infections récurrentes, les infections récurrentes chez l'enfant infecté par le VIH , le traitement de déficits immunitaires primitifs avec hypogammaglobulinémie ou atteinte fonctionnelle de l'immunité humorale, le traitement de déficits immunitaires secondaires de l'immunité humorale, en particulier la leucémie lymphoïde chronique ou le myélome avec hypogammaglobulinémie et associés à des infections à répétition, l'allogreffe de cellules souches hématopoïétiques avec hypogammaglobulinémie associée à une infection, le traitement immunomodulateur du purpura thrombopénique idiopathique (PTI) chez l'adulte et l'enfant en cas de risque hémorragique important ou avant un acte médical ou chirurgical, de la rétinochoroïdite de Birdshot, du syndrome de Guillain-Barré, de la neuropathie motrice multifocale (NMM), des polyradiculonévrites inflammatoires démyélinisantes chroniques (PIDC), et de la maladie de Kawasaki.

## Patentansprüche

1. Verfahren zur Herstellung eines Konzentrats polyvalenter menschlichen Immunglobuline anhand einer Ausgangslösung aus Blutplasma oder aus mit Immunglobulinen angereicherter Plasmafraktion, **dadurch gekennzeichnet, dass** es umfasst:
(a) einen Arbeitsschritt der Beseitigung der Proteinverunreinigungen durch die Caprylsäure zur Gewinnung einer proteasefreien Lösung, in welcher die eingesetzte Caprylsäure-Konzentration 0,5 bis 1,5 % beträgt und in Gramm Caprylsäure pro Volumen von zu verarbeitender Lösung angegeben ist,
(b) einen Arbeitsschritt der Klärung zu niedrigem pH-Wert durch Tiefenfiltration,
(c) gefolgt von einem Arbeitsschritt der Wirbelschicht-Chromatographie der proteasefreien Lösung,
wobei mit dem Verfahren ein Konzentrat polyvalenter menschlichen Immunglobuline mit einer Ausbeute von über 4,5 g Immunglobuline pro Liter verarbeiteten Blutplasmas gewonnen wird.

2. Verfahren nach Anspruch 1, **dadurch gekennzeichnet, dass** die verwendete Caprylsäure-Konzentration 0,8 bis 1,2 % beträgt und in Gramm Caprylsäure pro Volumen von zu verarbeitender Lösung angegeben ist.

3. Verfahren nach Anspruch 2, **dadurch gekennzeichnet, dass** die verwendete Caprylsäure-Konzentration 0,9 bis 1,1 % beträgt und in Gramm Caprylsäure pro Volumen von zu verarbeitender Lösung angegeben ist.

4. Verfahren nach einem der Ansprüche 1 bis 3, **dadurch gekennzeichnet, dass** der Arbeitsschritt der Beseitigung der Proteinverunreinigungen durch die Caprylsäure (a) zu einem pH-Wert zwischen 4,3 und 4,9, bevorzugt zwischen 4,6 und 4,8 erfolgt.

5. Verfahren nach einem der Ansprüche 1 bis 4, **dadurch gekennzeichnet, dass** der Arbeitsschritt der Wirbelschicht-Chromatographie der proteasefreien Lösung (c) mit einem chromatographischen Träger vom Typ Ionenaustausch, vom Typ Affinität, vom Typ "Pseudo-Affinität" oder vom Typ "Mischmodus" ausgeführt wird.

6. Verfahren nach Anspruch 5, wobei der Arbeitsschritt der Wirbelschicht-Chromatographie vom Typ "Mischmodus" umfasst:
- die Aufladung einer Chromatographiesäule, welche vorab mittels eines Puffers zu einem pH-Wert zwischen 4,5 und 8 eingestellt wurde, mit der vorab zu dem gleichen pH-Wert angepassten Lösung, die den Arbeitsschritt der Klärung durch Tiefenfiltration unterzogen wurde,
- die Bereinigung der genannten Säule mittels einer Pufferlösung, bis alle auf der Säule nicht adsorbierten Proteine beseitigt sind,
- die Elution der auf der Säule adsorbierten polyvalenten Immunglobuline mittels eines zu einem pH-Wert zwischen 8 und 10 eingestellten Elutionspuffers und
- die Rückgewinnung der mit menschlichen polyvalenten Immunglobulinen angereicherten Lösung.

7. Verfahren nach einem der Ansprüche 1 bis 6, nach dem Arbeitsschritt (c) außerdem einen oder mehrere der folgenden Arbeitsschritte umfassend:
(i) einen Arbeitsschritt der Virusinaktivierung,
(ii) einen Arbeitsschritt der Anionenaustauschchromatographie der am Ausgang des vorangegangenen Arbeitsschrittes gewonnenen Lösung,
(iii) einen Arbeitsschritt der Beseitigung von anti-A und anti-B Antikörpern aus der am Ausgang des vorangegangenen Arbeitsschrittes gewonnenen Lösung,
(iv) einen Arbeitsschritt der Filtration durch Nanometer-Filter mit absteigender Porosität von 100 auf 15 nm,
(v) einen Arbeitsschritt der Konzentration durch Ultrafiltration der Lösung aus dem vorangegangenen Arbeitsschritt, kombiniert mit einem Arbeitsschritt der Zusammensetzung,
(vi) anschließend einen Arbeitsschritt der herkömmlichen Entkeimungsfiltration.

8. Verfahren nach einem der Ansprüche 1 bis 7, wobei es sich bei der Ausgangslösung um eine Plasmafraktion handelt, welche durch Ethanol-Fraktionierung und/oder durch chromatographische Trennung mit Immunglobulinen angereichert wird.

9. Verfahren nach Anspruch 8, wobei die Ausgangslösung ein Niederschlag "I+II+III" oder ein Niederschlag "II+III" ist, der auf der Grundlage von mit Ethanol fraktioniertem Blutplasma gewonnen und in Lösung wieder gebracht wird.

10. Verfahren nach Anspruch 9, wobei der Niederschlag "I+II+II" oder der Niederschlag "II+II" in gereinigtem Wasser für eine Injektion oder in einer ionenhaltigen Lösung in Lösung wieder gebracht wird.

11. Verfahren nach Anspruch 10, wobei die ionenhaltige Lösung eine Lösung ist, die NaCl in einer Konzentration kleiner oder gleich 20 mM, vorzugsweise zwischen 5 und 15 mM und bevorzugt gleich 10 mM enthält.

12. Verfahren nach einem der Ansprüche 8 bis 11, wobei der Niederschlag "I+II+III" oder der Niederschlag "II+III" mit einer CaCl₂-Lösung, deren Konzentration kleiner oder gleich 20 mM ist, vorzugsweise zwischen 5 und 15 mM liegt, und bevorzugt mit einer CaCl₂-Lösung von 10 mM behandelt wird.

13. Verfahren nach einem der Ansprüche 1 bis 12, umfassend:
(i) einen Arbeitsschritt der Beseitigung der Proteinverunreinigungen durch Caprylsäure,
(ii) einen Arbeitsschritt der Klärung durch Tiefenfiltration,
(iii) einen Arbeitsschritt der Wirbelschicht-Chromatographie vom Typ "Mischmodus",
(iv) einen Arbeitsschritt der Virusinaktivierung durch Behandlung mit Lösungsmittel/ Detergens,
(v) einen Arbeitsschritt der Anionenaustauschchromatographie,
(vi) einen Arbeitsschritt der Beseitigung der anti-A- und anti-B-Antikörper,
(vii) eine Filtration durch Nanometer-Filter mit absteigender Porosität von 100 auf 15 nm,
(viii) einen Arbeitsschritt der Konzentration durch Ultrafiltration der Lösung aus dem vorangegangenen Arbeitsschritt, kombiniert mit einem Arbeitsschritt der Zusammensetzung, dann einen Arbeitsschritt der herkömmlichen Entkeimungsfiltration.

14. Verfahren nach einem der vorhergehenden Ansprüche 1 bis 13, außerdem folgende Arbeitsschritte umfassend:
(a) das Hinzufügen eines oder mehrerer pharmazeutisch zulässigen Stabilisatoren dem Konzentrat menschlicher polyvalenten Immunglobuline, das nach einem der Ansprüche 1 bis 13 gewonnen wurde, und
(b) optional das Gefrieren oder die Gefriertrocknung der im vorangegangenen Arbeitsschritt gewonnenen pharmazeutischen Zubereitung,
sodass die pharmazeutische Zubereitung entweder in flüssiger, gefrorener oder gefriergetrockneter Form vorliegt.

15. Konzentrat menschlicher polyvalenten Immunglobuline, nach dem Verfahren eines der Ansprüche 1 bis 14 gewonnen , **dadurch gekennzeichnet, dass** das Konzentrat eine antikomplementäre Aktivität niedriger als 30 % aufweist.

16. Pharmazeutische Zusammensetzung menschlicher polyvalenten Immunglobuline, flüssig, gefroren oder gefriergetrocknet, hergestellt nach dem Verfahren nach einem der Ansprüche 1 bis 14 für eine Anwendung in der Behandlung von Pathologien aus der Gruppe bestehend aus den demyelinisierenden Polyneuropathien, der Multiple-Sklerose, dem Lambert-Eaton myasthenischen Syndrom, der Dermatomyositis, in einer Ersatztherapie bei primären Antikörpermangelsyndromen wie der kongenitalen Agammaglobulinämie und der kongenitalen Hypogammaglobulinämie, dem variablen Immundefektsyndrom, dem schweren kombinierten Immundefekt, dem Wiskott-Aldrich-Syndrom, dem Myelom oder der chronisch lymphatischen Leukämie mit schwerer sekundären Hypogammaglobulinämie und wiederholten Infektionen, den wiederholten Infektionen bei mit HIV-infizierten Kindern, in der Behandlung von primären Antikörpermangelsyndromen mit Hypogammaglobulinämie oder funktioneller Erkrankung der humoralen Immunität, in der Behandlung von sekundären Antikörpermangelsyndromen der humoralen Immunität, insbesondere von der chronisch lymphatischen Leukämie oder von dem Myelom mit Hypogammaglobulinämie und kombiniert mit wiederholten Infektionen, von dem Allotransplantat von hämatopoietischen Stammzellen mit Hypogammaglobulinämie kombiniert mit einer Infektion, in der immunomodulatorischen Behandlung der idiopathischen thrombozytopenischen Purpura bei Erwachsenen und Kindern im Falle eines schweren hämorrhagischen Risikos oder vor einem therapeutischen bzw. chirurgischen Eingriff, der Birdshot Retinochoroidopathie, des Guillain-Barré Syndroms, der multifokalen motorischen Neuropathie (MMN), der chronisch demyelinisierenden infektiösen Polyradikuloneuritis (CDIP) und des Kawasaki-Syndroms.

## Claims

1. A method for preparing a concentrate of human polyvalent immunoglobulins from an initial solution of blood plasma or a fraction of plasma enriched with immunoglobulins, **characterized in that** the method comprises :
(a) a step for removing protein contaminants with caprylic acid in order to obtain a solution free of proteases, wherein the concentration of caprylic acid used ranges from 0.5 to 1.5 %, expressed in grams of caprylic acid per volume of solution to be treated,
(b) a clarification step by depth filtration at acid pH,
(c) followed by a fluidized bed chromatography step for the solution free of proteases, said method allowing a concentrate of human polyvalent immunoglobulins with a yield of more than 4.5 g of immunoglobulins per liter of blood plasma applied to be obtained.

2. The method according to claim 1, **characterized in that** the concentration of caprylic acid used ranges from 0.8 to 1.2%, expressed in grams of caprylic acid per volume of solution to be treated.

3. The method according to claim 2, **characterized in that** the concentration of caprylic acid used ranges from 0,9 to 1,1%, expressed in grams of caprylic acid per volume of solution to be treated.

4. The method according to any one of claims 1 to 3, **characterized in that** the step for removing protein contaminants with caprylic acid (a) is carried out at a pH between 4.3 and 4.9, preferably comprised between 4.6 and 4.8.

5. The method according to any one of claims 1 to 4, **characterized in that** the fluidized bed chromatography step for the solution free of proteases (c) is carried out with a chromatographic support of the ion exchange type, of the affinity type of the "pseudo-affinity" type or of the "mixed-mode" type.

6. The method according to claim 5, wherein the fluidized bed chromatography step of the "mixed-mode" type comprises:
- loading on a chromatography column equilibrated beforehand with a buffer at a pH between 4.5 and 8, the solution having undergone the clarification step by depth filtration, adjusted beforehand to the same pH,
- washing the loaded column with a buffer solution until all the non-adsorbed proteins on the column are removed,
- eluting polyvalent immunoglobulins adsorbed on the column with an elution buffer adjusted to a pH between 8 and 10, and
- recovering the solution enriched with human polyvalent immunoglobulins.

7. The method according to any one of claims 1 to 6 further comprising, after step (c), one or more of the following steps:
(i) a viral inactivation step,
(ii) a step for anion exchange chromatography of the solution obtained at the end of the preceding step,
(iii) a step for removing anti-A and anti-B antibodies from the solution obtained at the end of the preceding step,
(iv) a filtration step through nanometric filters with decreasing porosity from 100 to 15 nm,
(v) a concentration step by ultrafiltration of the solution from the preceding step associated with a formulation step,
(vi) and then a conventional sterilizing filtration step.

8. The method according to any one of claims 1 to 7, wherein the initial solution is a plasma fraction enriched with immunoglobulins by fractionation with ethanol and/or by separation with chromatography.

9. The method according to claim 8, wherein the initial solution is a "I+II+III" precipitate or a "II+III" precipitate obtained from blood plasma fractionated with ethanol, and put back into solution.

10. The method according to claim 9, wherein the "I+II+III" precipitate or the "II+III" precipitate is put back into solution in purified water for injection or in a solution containing ions.

11. The method according to claim 10, wherein the solution containing ions is a solution comprising NaCl at a concentration of less than or equal to 20 mM, preferably comprised between 5 and 15 mM and preferably with a 10mM.

12. The method according to any one of claims 8 to 11, wherein the "I+II+III" precipitate or the "II+III" precipitate is treated with CaCl₂ solution, the concentration of which is less than or equal to 20 mM, preferably comprised between 5 and 15 mM and preferably with a 10mM CaCl₂ solution.

13. The method according to any one of claims 1 to 12, comprising:
(i) a step for removing protein contaminants with caprylic acid,
(ii) a clarification step by depth filtration,
(iii) a fluidized bed chromatography step of the "mixed-mode" type,
(iv) a viral inactivation step with solvent/detergent treatment,
(v) an anion exchange chromatography step,
(vi) a step for removing anti-A and anti-B antibodies,
(vii) a filtration through nanometric filters with decreasing porosity from 100 to 15 nm,
(viii) a step for concentrating by ultrafiltration the solution from the preceding step associated with a formulation step, and then a conventional sterilizing filtration step.

14. The method according to any one of claims 1 to 13, further comprising the following steps:
(a) adding to the concentrate of human polyvalent immunoglobulins obtained according to any one of claims 1 to 13, one or several pharmaceutically acceptable stabilizers, and
(b) optionally, freezing or freeze-drying the pharmaceutical preparation obtained in the preceding step;
so that the pharmaceutical preparation is in a liquid, frozen or freeze-dried form.

15. A concentrate of human polyvalent immunoglobulins obtained according to the method of any one of claims 1 to 14, **characterized in that** said concentrate has an anti-complementary activity of less than 30%.

16. A liquid, frozen or freeze-dried pharmaceutical composition of human polyvalent immunoglobulins obtained according to the method of any one of claim 1 to 14, for its use in the treatment of pathologies comprised in the group consisting of demyelinating polyneuropathies, multiple sclerosis, myasthenic Lambert-Eaton syndrome, dermatomyositis, a substitutive treatment in the case of primary immunodeficiencies such as congenital agammaglobulinemias and congenital hypogammaglobulinemias, variable common immunodeficiency, severe combined immunodeficiency, Wiskott Aldrich syndrome, myeloma or chronic lymphoid leukemia with severe secondary hypogammaglobulinemia and recurrent infections, recurrent infections in HIV-infected children, treatment of primary immunodeficiencies with hypogammaglobulinemia or functional impairment of humoral immunity, treatment of secondary immunodeficiency of humoral immunity, in particular chronic lymphoid leukemia or myeloma with hypogammaglobulinemia and associated with recurrent infections, allografting of hematopoietic stem cells with hypogammaglobulinemia associated with an infection, immunomodulating treatment of idiopathic thrombocytopenic purpura (ITP) in adults and children in the case of significant hemorrhagic risk or before a medical or surgical operation, of Birdshot's retinochoroiditis, Guillain-Barré syndrome, multifocal motor neuropathy (MMN), chronic inflammatory demyelinating polyneuropathies (CIDP), or Kawasaki's disease.
